# EUROPEAN PATENT APPLICATION

(11) **EP 3 730 161 A1**
(43) Date of publication of application: **28.10.2020**
(21) Application number: 19849012.0
(22) Date of filing: 08.03.2019
(51) Int. Cl.: A61L 27/26, A61L 27/20, A61L 27/58, A61F 2/00

(54) **METHOD FOR PRODUCING COMPLEX**

(71) Applicant: GCS Co., Ltd., Seongnam-si, Gyeonggi-do 13215 (KR)
(72) Inventor: KIM, Chang-Sik, Suwon-si Gyeonggi-do 16557 (KR)
(74) Representative: Fuchs Patentanwälte Partnerschaft mbB
(86) International application number: PCT/KR2019/002765
(87) International publication number: WO 2020/184744

(57) **Abstract**

Disclosed is a method of preparing a composite, particularly, a composite containing a poly-L-lactic acid (PLLA) filler and a hyaluronic acid (HA) filler. The composite prepared by the method according to the present application is capable of minimizing the occurrence of defects such as nodules or granulomas. The composite prepared by the method of the present application has an advantage of maintaining the effect of the volume increase after the injection for a long time. Also, the method according to the present application has an advantage of preparing HA fillers applied to the composite at high purity.

## Description

### [Technical Field]

The present invention relates to a method of preparing a composite and more particularly to a method of preparing a composite containing a poly-L-lactic acid (PLLA) filler and a hyaluronic acid (HA) filler.

### [Background Art]

Poly-L-lactic acid (PLLA) fillers, specifically PLLA dermal fillers, are generally known biocompatible and biodegradable polymers and serve as supports of microspheres. Hyaluronic acid (HA) is a biosynthetic natural substance extracted mainly from the skin of animals and the like. HA fillers, specifically HA dermal fillers, are mainly used to increase the volume of the skin of the facial area by non-surgical therapy.

Conventional PLLA fillers were prepared and injected in the following manner. First, PLLA in the form of a dried powder is mixed with carboxymethylcellulose (CMC) and mannitol, and is then lyophilized. The lyophilized PLLA is then mixed with sterile distilled water to prepare a suspension. Then, the suspension is injected into the human body using a syringe or the like. When the suspension is injected into the body, collagen is produced in the body at least 3 to 6 months after the injection, providing an effect of increasing the volume of the injection zone.

However, in the prior art, since the ingredients of distilled water and CMC in the suspension injected into the human body are absorbed and disappear in the body within a few days, there is a problem in that the effect of increase in the area where the suspension is injected occurs at least several months (3 to 6 months) after injection into the body. As such, it takes several months after the injection into the body for collagen production, which is the main cause of users' (subjects) complaints.

In order to solve this problem, the use of different kinds of biomaterials for restoring human body in combination has been considered in order for PLLA particles to be uniformly distributed in the body and to be maintained at a constant volume until collagen is produced after the injection of the PLLA filler. However, the use of different kinds of biomaterials in combination in this way also causes a problem of inconvenience (complication) of the procedure. In addition, conventional PLLA fillers have a disadvantage of causing defects such as nodules or granulomas.

In addition, a suspension of PLLA fillers diluted with distilled water is injected into a subject. Here, there is a problem in that the concentration of the suspension is lowered when mixed with distilled water. For this reason, disadvantageously, the mixing should be conducted for a long time to obtain a homogeneous suspension.

Another attempt to solve the problem involves synthesizing PLLA in a unique manner. Specifically, there has been suggested PLLA polymerization that includes, without preparing lactide, which is a cyclic dimer of lactic acid, conducting dehydration-condensation of an oligomer of L-lactic acid {oligo(L-lactic acid)} in the absence of a catalyst, adding a certain ratio of an Sn compound and a proton acid as a catalyst, conducting direct dehydration-polymerization under reduced pressure to synthesize a low-molecular-weight PLLA, preheating the PLLA to increase the crystallinity and conducting solid-phase polymerization thereon. However, disadvantageously, this method was incapable of sufficiently solving the conventional problems.

HA fillers are generally prepared by reacting HA with a cross-linking agent such as butanediol diglycidyl ether. Specifically, a method including mixing a crosslinking agent with HA in the presence of an aqueous alkali solution and reacting the resulting mixture in a liquid phase has been applied. In this method, an excess of crosslinking agent is added for sufficient crosslinking reaction of HA, and the added crosslinking agent should be sufficiently removed during the purification process since the majority of crosslinking agents cause toxicity in the body. However, this method has a problem in that removal thereof is difficult. In addition, the HA fillers prepared as described above have relatively low stability to withstand HA-degrading enzymes or heat and difficulty in removing unreacted substances, thus having limited usefulness as high-purity biocompatible materials.

### [Disclosure]

### [Technical Problem]

Therefore, the present invention has been made in view of the above problems, and it is one object of the present invention to provide a method for preparing a composite that is capable of minimizing the occurrence of defects such as nodules or granulomas, maintaining an increase in volume after injection, and preparing the composite with high purity without adding an excessive amount of a crosslinking agent.

### [Technical Solution]

In accordance with the present invention, the above and other objects can be accomplished by the provision of a method of preparing a composite including crosslinking a mixture of hyaluronic acid (HA) and a crosslinking agent in a solid phase in the presence of an organic solvent to prepare a HA filler; freeze-drying a mixture containing poly-L-lactic acid (PLLA), carboxymethylcellulose and mannitol to prepare a PLLA filler and mixing the HA filler with the poly-L-lactic acid (PLLA) filler.

### [Advantageous effects]

The composite, more specifically, a composite containing a poly-L-lactic acid (PLLA) filler and a hyaluronic acid (HA) filler, prepared by the method according to the present application, is capable of minimizing the occurrence of defects such as nodules or granulomas.

The composite prepared by the method of the present application has an advantage of maintaining the effect of the volume increase after the injection for a long time.

The method according to the present application has an advantage of preparing HA fillers applied to the composite of the present application at high purity.

### [Best Mode]

The method of preparing a composite according to the present application includes crosslinking a mixture of hyaluronic acid (HA) and a crosslinking agent in a solid phase in the presence of an organic solvent to prepare a HA filler, freeze-drying a mixture containing poly-L-lactic acid (PLLA), carboxymethylcellulose and mannitol to prepare a PLLA filler, and mixing the HA filler with the poly-L-lactic acid (PLLA) filler.

In addition, the method of preparing a composite according to the present application may further include stirring after the mixing.

### [Mode for Invention]

The terms used in the present application may be interpreted as having meanings commonly used in the art unless otherwise specified in the present specification.

The physical properties of certain compounds mentioned in the present application can be measured by a method commonly used in the art, unless otherwise specified herein.

The present application relates to a method for preparing a composite, specifically, a composite containing a poly-L-lactic acid (PLLA) filler and a hyaluronic acid (HA) filler. In the method, a composite of certain substances may mean that the substances are independently present in one composite, or are bonded or crosslinked by a physical or chemical method.

The method of the present application may include crosslinking a mixture of hyaluronic acid (HA) and a crosslinking agent to prepare a HA filler. Specifically, the HA filler may be prepared by crosslinking the mixture in a solid phase in the presence of an organic solvent.

In one example, hyaluronic acid may include both hyaluronic acid itself and a salt thereof. The salt of hyaluronic acid may include all metal salts or organic salts of hyaluronic acid. The metal salt of hyaluronic acid may be, for example, sodium hyaluronate, potassium hyaluronate, calcium hyaluronate, magnesium hyaluronate zinc hyaluronate, or cobalt hyaluronate. The metal salt of hyaluronic acid may be, for example, tetrabutylammonium or the like.

The molecular weight of the hyaluronic acid is not particularly limited, but may be, for example, in the range of 100000 to 5000000. In another example, the molecular weight is 200000 or more, 300000 or more, 400000 or more, 500000 or more, 600000 or more, 700000 or more, 800000 or more, 900000 or more, 1000000 or more, 1100000 or more, 1200000 or more, 1300000 or more, 1400000 or more, 1500000 or more, 1600000 or more, 1700000 or more, 1800000 or more, 1900000 or mor or 2000000 or more, and 4000000 or less, 3000000 or less, 2900000 or less, 2800000 or less, 2700000 or less, 2600000 or less, 2500000 or less, 2400000 or less, 2300000 or less, 2200000 or less, 2100000 or less, or 2000000 or less. The unit of molecular weight may be "Da (daltons)".

The molecular weight can be measured using a well-known method. For example, the molecular weight may be determined by measuring a limiting viscosity at the kinematic viscosity of the hyaluronic acid and converting the limiting viscosity into a molecular weight. In another example, the molecular weight may be measured by a simple measurement method using liquid chromatography.

In one example, the crosslinking agent may be a compound having at least two epoxy groups. Specifically, the crosslinking agent may include at least one of 1,4-butanediol diglycidyl ether (BDDE), ethylene glycol diglycidyl ether (EGDGE), 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycol polyglycidyl ether, diglycerol polyglycidyl ether, glycerol polyglycidyl ether, tri-methylpropane polyglycidyl ether, 1,2-(bis(2,3-epoxypropoxy) ethylene, pentaerythritol polyglycidyl ether and sorbitol polyglycidyl ether, and is more preferably butanediol diglycidyl ether.

The HA filler may be formed by crosslinking an epoxy group in the crosslinking agent with an alcohol group of HA. The epoxy group in the crosslinking agent reacts with a hydroxyl group in HA to form an ether bond, and the reaction of the epoxy group with the hydroxyl group occurs very well. Thus, the HA filler can be prepared with a high yield using the crosslinking agent.

The crosslinking reaction of the mixture including the HA and the crosslinking agent may be performed in a solid phase. Specifically, the crosslinking reaction may be performed by dispersing the mixture in a solid phase in an organic solvent and then inducing a reaction between the HA and the crosslinking agent.

The organic solvent is preferably a substance that does not mix well with the mixture in order for the mixture to be present as a solid, rather than being dissolved in the solvent, when the organic solvent is mixed with the mixture. For example, acetone, an aqueous solution of acetone, an alcohol, or an aqueous solution of alcohol may be used as the solvent. The alcohol may be alcohol having 2 to 10 carbon atoms, 2 to 8 carbon atoms, or 2 to 6 carbon atoms.

The reaction between HA and the crosslinking agent in a liquid phase may cause a problem of low reaction yield. At this time, functional groups, in particular, epoxy groups, in the crosslinking agent, remain in the mixture, which may react with other substances present in the body. Therefore, the crosslinking reaction in a liquid phase requires an additional process of extremely accurately metering the crosslinking agent before addition, further conducting subsequent purification to minimize the content of remaining functional groups, or hydrolyzing the functional groups and converting the same into inert functional groups.

The step of preparing the HA filler can be specified as follows:

(1) HA is dissolved in an aqueous alkali solution.

(2) a crosslinking agent is added to the aqueous alkali solution in which HA is dissolved to prepare a mixture, and the mixture is homogeneously stirred.

(3) The stirred mixture is added dropwise to an organic solvent, and the mixture of the hyaluronic acid and the crosslinking agent is subjected to cross-linking reaction in a solid phase.

When the HA is dissolved in the aqueous alkali solution, the hyaluronic acid may be dissolved in an amount of 1% to 50%, or 5% to 15%, based on the total weight of solids in the solution.

The type of alkali compound applied to the aqueous alkali solution is not particularly limited. For example, the alkali compound is a known basic compound such as sodium hydroxide, potassium hydroxide or ammonia water, and is preferably sodium hydroxide. The concentration of the aqueous alkali solution is not particularly limited, and may be, for example, within the range of 0.1M to 10M.

In step (3), a salt such as sodium chloride may be further added to the aqueous alkali solution in which HA is dissolved in order to reduce the solubility of HA in the organic solvent and thereby to facilitate conversion into a solid phase. In this case, the appropriate concentration of the salt is in the range of 1 mg/mL to 1,000 mg/mL and the amount thereof is not particularly limited.

The mix ratio of HA and the crosslinking agent is not specifically limited, but is for example within the range of 0.01 equivalent% to 50 equivalent%, or 0.1 equivalent% to 150 equivalent%, per repeating unit of HA.

The crosslinking reaction of the mixture may be carried out within a temperature range of 15°C to 60°C or 25°C to 50°C. The period of time of the crosslinking reaction is not particularly limited. As the reaction time increases, the HA is decomposed, thus causing a problem of deterioration in the physical viscoelasticity of the HA filler. Thus, the crosslinking reaction is preferably carried out for 1 hour to 48 hours.

The preparation process of the HA filler may further include washing the mixture after the crosslinking reaction between the HA and the crosslinking agent to remove the unreacted crosslinking agent or the residue. The washing may be carried out using distilled water, a buffer solution, or an aqueous solution added with a salt.

The buffer solution may be an acidic solution such as hydrochloric acid, sulfuric acid, nitric acid, phosphoric acid or acetic acid. However, a phosphoric acid buffer solution is preferably used in consideration of washing efficiency.

The amount of the phosphoric acid buffer solution may be within the range of 30L to 80L with respect to 100 g of HA. In another example, the amount that is used is 35L or more, 40L or more, 45L or more, 50L or more, and 75L or less, 70L or less, 65L or less, 60L or less, 55L or less or 50L or less.

The method of obtaining the HA filler is not particularly limited. For example, the HA filler may be obtained by filtering the washed mixture. For example, the HA filler may be obtained in the form of a particle having a uniform size by passing the washed mixture through a filter with a constant size.

In this case, the size of the filter that is applied may be 80 mesh to 120 mesh. In another example, the specification may be 85 mesh or more, 90 mesh or more, 95 mesh or more or 100 mesh or more, and 115 mesh or less, 110 mesh or less, 105 mesh or less, or 100 mesh or less.

The obtained HA may be filled in a vial. In addition, the HA filling the vial may be subjected to further treatment such as high-pressure sterilization.

The method for preparing a composite of the present application includes drying the mixture including poly-L-lactic acid (PLLA), carboxymethylcellulose and mannitol to prepare a PLLA filler. Specifically, the preparation method of the present application may include freeze-drying the mixture to prepare a PLLA filler.

In the present application, the term "freeze drying", as defined, may refer to a method including freezing a material containing a large amount of moisture and then drying the material through a process of sublimating ice under reduced pressure to remove the moisture therefrom.

The initial freezing temperature of the freeze drying may be within the range of -100°C to -60°C. In addition, the PLLA filler may be prepared by freezing the mixture including PLLA and the like within the initial freezing temperature range and then drying the mixture at a temperature within a range of 15°C to 25°C. The time required for the drying is not particularly limited, but, for example, the frozen PLLA mixture may be dried from a period of time of 5 days to 10 days.

The freeze-dried PLLA mixture may be pulverized to prepare a PLLA filler in the form of a particle. The pulverization method is not particularly limited, and a general overhead stirrer may be applied.

The shape of the particle is also not particularly limited. The two-dimensional shape of the particle may be a curved shape such as a circle or an ellipse, a polygon such as a triangle, a square or a pentagon, or may be amorphous.

The average size of the PLLA filler in the form of a pulverized particle may be within the range of 30 µm to 100 µm. In another example, the size may be 35 µm or more, 40 µm or more, 45 µm or more or 50 µm or more, and 95 µm or less, 90 µm or less, 85 µm or less, 80 µm or less, 75 µm or less, 70 µm or less, 65 µm or less, 60 µm or less, 55 µm or less or 50 µm or less.

The pulverized particle-form PLLA filler may be contained in an appropriate amount in a vial. In addition, the PLLA filler contained in the vial may be subjected to gamma-ray sterilization. Gamma-ray sterilization is a known radiation sterilization method, and may refer to a treatment method for removing contaminants such as microorganisms from the corresponding material using gamma rays, typically generated from cobalt.

The method for preparing a composite of the present application includes mixing the PLLA filler with the HA filler. Specifically, the mixing may be carried out after injecting the HA filler into the vial containing the PLLA filler. In addition, the HA filler may be mixed with distilled water before injecting the HA filler into the vial containing the PLLA filler. The PLLA filler and the HA filler mixed as described above may be extracted using a syringe and then injected into a subject.

The mixing ratio of distilled water may vary depending on the site where treatment with the composite occurs.

When the composite is injected into the face of the subject, the ratio (V_{w}/V_{HA}) of the volume (V_{w}) of the distilled water to the volume (V_{HA}) of the HA filler may be within the range of 1.5 to 2.5. In another example, the ratio may be 1.6 or more, 1.7 or more, 1.8 or more, 1.9 or more or 2.0 or more, and 2.4 or less, 2.3 or less, 2.1 or less or 2.0 or less.

When the composite is injected into a portion of the body other than the user's (subject's) face, the ratio (V_{w}/V_{HA}) of the volume (V_{w}) of the distilled water to the volume (V_{HA}) of the HA filler may be in the range of 2.5 to 3.5. In another example, the ratio may be 2.6 or more, 2.7 or more, 2.8 or more, 2.9 or more or 3.0 or more, and 3.4 or less, 3.3 or less, 3.2 or less, 3.1 or less or 3.0 or less.

The method of preparing a composite of the present application may further include stirring the mixture of the PLLA filler and the HA filler after mixing the mixture and before injecting the mixture into the subject. The stirring method is not particularly limited and may be carried out by shaking the PLLA-containing vial, into which the HA filler is injected, by hand or using a stirring device for a predetermined time prior to treatment.

The stirring time is not particularly limited, and is, for example, 3 minutes or more, 3.5 minutes or more, 4 minutes or more, 4.5 minutes or more or 5 minutes or more, and 8 minutes or less, 7.5 minutes or less, 7 minutes or less, 6.5 minutes or less, 6 minutes or less, 5.5 minutes or less or 5 minutes or less.

The composite prepared through the steps descried above, specifically, the composite containing the PLLA filler and the HA filler, can minimize the occurrence of defects such as nodules or granulomas, greatly shorten the time required to increase the volume of the injection site after injection, and maintain the effect of the volume increase for a long time.

## Claims

1. A method of preparing a composite comprising:
crosslinking a mixture of hyaluronic acid (HA) and a crosslinking agent in a solid phase in presence of an organic solvent to prepare a HA filler;
freeze-drying a mixture containing poly-L-lactic acid (PLLA), carboxymethylcellulose and mannitol to prepare a PLLA filler; and
mixing the HA filler with the poly-L-lactic acid (PLLA) filler.

2. The method according to claim 1, wherein the hyaluronic acid comprises at least one of hyaluronic acid or a salt of hyaluronic acid.

3. The method according to claim 1, wherein the crosslinking agent is a compound having at least two epoxy groups.

4. The method according to claim 3, wherein the crosslinking agent comprises at least one of 1,4-butanediol diglycidyl ether, ethylene glycol diglycidyl ether, 1,6-hexanediol diglycidyl ether, propylene glycol diglycidyl ether, polypropylene glycol diglycidyl ether, polytetramethylene glycol diglycidyl ether, neopentyl glycol diglycidyl ether, polyglycol polyglycidyl ether, diglycerol polyglycidyl ether, glycerol polyglycidyl ether, tri-methylpropane polyglycidyl ether, 1,2-(bis(2,3-epoxypropoxy) ethylene, pentaerythritol polyglycidyl ether and sorbitol polyglycidyl ether.

5. The method according to claim 4, wherein the crosslinking agent is 1,4-butanediol diglycidyl ether.

6. The method according to claim 1, wherein the organic solvent is acetone, an alcohol, or an aqueous solution of at least one thereof.

7. The method according to claim 1, wherein the HA filler is preparing by washing the cross-linked mixture with a buffer solution.

8. The method according to claim 7, wherein an amount of the buffer solution that is used is within a range of 30L to 80L with respect to 100 g of the HA.

9. The method according to claim 7, further comprising filtering the mixture through an 80 mesh to 120 mesh filter after the washing.

10. The method according to claim 7, further comprising sterilizing the mixture under high pressure after the washing.

11. The method according to claim 1, wherein the freeze-drying comprises freezing the mixture containing poly-L-lactic acid (PLLA), carboxymethylcellulose and mannitol at a temperature of -100°C to -60°C.

12. The method according to claim 11, wherein the freeze-drying further comprises drying the frozen mixture at a temperature of 15°C to 25°C for a period of time of 5 days to 10 days, after the freezing.

13. The method according to claim 1, wherein the preparing a PPL filter further comprises pulverizing the freeze-dried mixture to prepare a PLLA filler in a form of a particle.

14. The method according to claim 13, wherein an average size of the particle-form PLLA filler is within a range of 30 µm to 100 µm.

15. The method according to claim 1, wherein the preparing a PLLA filler further comprises sterilizing the freeze-dried mixture with gamma rays after the freeze-drying.

16. The method according to claim 1, wherein the mixing comprises mixing the HA filler with the PLLA filler after mixing the HA filler with distilled water.

17. The method according to claim 15, wherein a mixing ratio (V_{w}/V_{HA}) of a volume (V_{w}) of the distilled water to a volume (V_{HA}) of the HA filler is within a range of 1.5 to 2.5.

18. The method according to claim 15, wherein a mixing ratio (V_{w}/V_{HA}) of a volume (V_{w}) of the distilled water to a volume (V_{HA}) of the HA filler is within a range of 2.5 to 3.5.

19. The method according to claim 1, further comprising stirring the mixture for a period of time of 3 to 8 minutes after the mixing.
